# EUROPEAN PATENT SPECIFICATION

(11) **EP 1 531 851 B1**
(45) Date of publication and mention of the grant of the patent: **14.12.2011**
(21) Application number: 03757380.5
(22) Date of filing: 06.06.2003
(51) Int. Cl.: A61K 47/48, A61K 38/00, A61K 38/40, A61P 35/00, A61P 31/04

(54) **COVALENT CONJUGATES BETWEEN ARTEMISININ-RELATED ENDOPEROXIDES AND IRON-CARRYING PROTEINS AND METHODS OF USE**
KOVALENTE KONJUGATE ZWISCHEN MIT ARTEMISININ VERWANDTEN ENDOPEROXIDEN UND EISENTRAGENDEN PROTEINEN UND ANWENDUNGSVERFAHREN
CONJUGUES COVALENTS ENTRE DES ENDOPEROXYDES LIES A L'ARTEMISININE ET DES PROTEINES PORTEUSES DE FER ET PROCEDES D'UTILISATION ASSOCIES

(30) Priority: 06.06.2002 US 386928 P
(43) Date of publication of application: 25.05.2005
(73) Proprietor: University of Washington, Seattle, WA 98105-4608 (US)
(72) Inventor: LAI, Henry, C., Seattle, WA 98105 (US); SASAKI, Tomikazu, Bothell, WA 98012 (US); SINGH, Narendra, P., Lynnwood, WA 98037 (US)
(74) Representative: Bizley, Richard Edward
(86) International application number: PCT/US2003/017881
(87) International publication number: WO 2003/103592

(56) References cited:
- WO-A-96/34602
- WO-A-02/091992
- US-A- 5 578 637
- US-A- 6 127 405
- US-B1- 6 214 864
- LAI H. AND SINGH N.P.: "Selective cancer cell cytotoxicity from exposure to dihydroartemisinin and holotransferrin" CANCER LETTERS, vol. 91, no. 1, 4 May 1995 (1995-05-04), pages 41-46, XP002457448
- SINGH N.P. AND LAI H.: "Selective toxicity of dihydroartemisinin and holotransferrin toward human breast cancer cells" LIFE SCIENCES, vol. 70, no. 1, November 2001 (2001-11), pages 49-56, XP002457449
- SADAVA D. ET AL.: "Transferrin overcomes drug resistance to artemisinin in human small-cell lung carcinoma cells" CANCER LETTERS, vol. 179, no. 2, 28 May 2002 (2002-05-28), pages 151-156, XP002457450

## Description

### FIELD OF THE INVENTION

The invention relates to covalent conjugates between artemisinin-related endoperoxides and iron-carrying proteins according to the claims for use in a method of treating and infections caused by pathogens that bind iron-carrying proteins.

### BACKGROUND OF THE INVENTION

Artemisinin is a sesquiterpene lactone isolated from the plant *Artemisia annua* L, extracts of which has been used to treat malaria for at least 1600 years. The artemisinin molecule contains an endoperoxide bridge that reacts with an iron atom to form free radicals. The anti-malarial action of artemisinin is due to its reaction with intra-parasitic heme to generate free radicals, causing cell death. Cancer cells have a significantly higher influx of iron than normal cells. Accordingly, it has been shown that artemisinin and artemisinin analogs are cytotoxic against established tumors and tumor cell lines (*see, e.g*., Woerdenbag et al. (1993) J. Nat. Prod. 56(6):849-56; Lai & Singh (1995) Cancer Lett. 91:41-6*;* Efferth et al. (2001) Int. J. Oncol. 18:767-73; Li et al. (2001) Bioorg. Mend Chem. Lett. 11:5-8; Singh & Lai (2001) Life Sci. 70:49-56; Efferth et al. (2002) Biochem. Pharmacol. 64:617-23; Efferth et al. (2002) Blood Cells, Molecules & Diseases 28(2): 160-8; Sadava et al. (2002) Cancer Lett. 179:151-6).

Many analogs of artemisinin and other compounds containing an endoperoxide bridge that are biologically active have been described *(see, e.g.,* U.S. Patent No. 5,180,840; U.S. Patent No. 5,216,175; U.S. Patent No. 5,225,427; Cumming et al. (1998) J. Med. Chem. 41(6):952-64; Posner et al. (1999) J. Med. Chem. 42:300-4; Li et al. (2001) Bioorg. Med Chem. Lett. 11:5-8; Wu et al. (2001) Eur. J. Med Chem. 36:469-79; Posner et al. (2003) J. Med Chem 46:1060-5). Analogs of artemisinin that have been used in the treatment of malaria include dihydroartemisinin, artemether, artesunate, arteether, propylcarbonate dihydroartemisinin and artelinic acid.

Artemisinin is a relatively safe drug with few and minor side effects even at high doses. Oral doses of 70 mg/kg/day for 6 days has been used in humans for malaria treatment. No apparent adverse side effects were observed after treatment of a cancer patient with artesunate (oral dose of 50 mg per day; intramuscular dose of 60 mg/day, for a period of 9 months) (Singh & Verma (2002) Arch. Oncol. 10(4):279-80). Artemisinin and artemisinin analogs have also been used in the treatment of skin conditions such as psoriasis, blistering skin diseases, viral warts, mulluscum contagiosum, and hemorrhoids *(see, e.g.,* U.S. Patent No. 4,978,676; U.S. Patent No. 5,219,880). Artemisinin and artemisinin analogs have also been used for malaria prophylaxis.

It has been shown that administration of iron salts or the iron-carrying protein holotransferrin increases the susceptibility of cancer cells and implanted tumors to artemisinin and its analogs (Lai & Singh (1995) Cancer Lett. 91:41-46; Moore et al. (1995) Cancer Lett. 98:83-7; Singh & Lai (2001) Life Sci. 70:49-56; Sadava et al. (2002) Cancer Lett. 1179:151-6).

It has also been shown that certain pathogens obtain iron by binding to iron-carrying host proteins. For example, *Neisseria meningitidis,* the causative agent of bacterial meningitis, expresses cell surface receptors for iron-carrying compounds such as transferrin and lactoferrin (Evans & Oakhill (2002) Biochem. Soc. Trans. 30(4):705-7). Currently, no vaccine is available for the B strain of *N. meningitidis,* the most prevalent strain in the Western world. Furthermore, it has been shown that *Helicobacter pylori,* the etiologic agent of gastritis, gastric and duodenal ulcers, and adenocarcinoma in humans, obtains iron by binding human lactoferrin (Husson et al. (1993) Infect. Immun. 61(6):2694-7).

WO 02/091992 discloses conjugates of transferrin or transcobalamin with anti-protozoan drugs useful in the treatment of protozoan infections.

There is a need in the art for artemisinin compositions with increased efficacy for the treatment of cancer and disease caused by pathogens that bind iron-carrying host proteins. There is also a need for methods for treating cancer and infections caused by pathogens that obtain iron by internalizing iron-carrying host proteins. The present invention addresses these needs.

### SUMMARY OF THE INVENTION

Described herein are new compounds and compositions comprising covalent conjugates between an artemisinin-related endoperoxide and an iron- carrying protein.

The invention provides a covalent conjugate consisting of an artemisinin-related endoperoxide selected from the group consisting of sesquiterpene lactones and alcohols, carbonates, esters, ethers, and sulfonates thereof, arteflene, 1,2,4-trioxanes, and 1,2,4,5-tetraoxanes covalently conjugated to an iron-carrying protein selected from the group consisting of transferrin, holotransferrin, lactoferrin, hololactoferrin, haemoglobin, hemopexin, and neutral gelatinase-associated lipocalin, wherein the iron-carrying protein releases iron that reacts with an endoperoxide bridge on the endoperoxide to form free radicals. The artemisinin-related endoperoxide may be linked to the iron-carrying compound by a hydrazide moiety, a hydrazine moiety, or an aminoxy moiety. The artemisinin-related endoperoxide may have the structure: 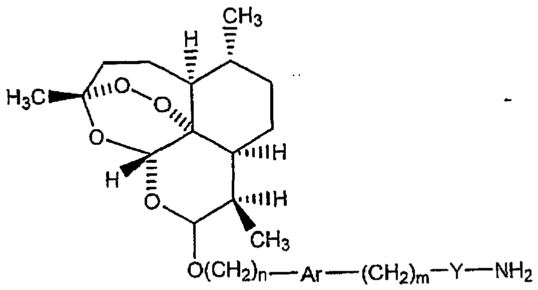 where n = 1-3, m = 0-3, Ar = aryl, and Y =- (C=O) NH-, -NH-, or-O-. Representative artemisinin-related endoperoxides present in the covalent conjugates of the invention include artelinate and dihydroartemisinin.

Representative iron-carrying proteins include the transferrin family of proteins, neutral gelatinase-associated lipocalin (NGAL) and hemoproteins. Thus, the covalent conjugates of the invention may comprise, for example, conjugates of artelinate and holotransferrin, artelinate and hololactoferrin, or artelinate and hemoglobin. The invention also provides compositions comprising the covalent conjugates of the invention and a pharmaceutically acceptable carrier. The covalent conjugates of the invention may be used in the treatment of cancer and infections by pathogens that bind iron-carrying proteins.

Described herein are the covalent conjugates of the invention for administration to a subject in need thereof. Exemplary covalent conjugates suitable for administration include, for example, covalent conjugates between artelinate and holotransferrin, between artelinate and hololactoferrin, and between artelinate and hemoglobin.

The invention provides covalent conjugates and compositions for use in the treatment of cancer, by administering to a human or animal subject in need thereof an effective amount of a composition comprising a covalent conjugate between an artemisinin-related endoperoxide and an iron-carrying protein according to the claims to a subject in need thereof. The covalent conjugates may be administered topically, systemically, or they may be injected directly into a tumor. The covalent conjugates may be administered alone or in combination with one or more additional therapeutic agents. For example, the covalent conjugates may be administered with an agent that increases the transport of iron into cells, for example by increasing the cell surface number of receptors for the iron-carrying proteins in the conjugate.

The invention also provides covalent conjugates and compositions for use in the treatment of infections by pathogens that bind to iron-carrying proteins, by administering to a human or animal subject in need thereof an effective amount of a composition comprising a covalent conjugate between an artemisinin-related endoperoxide and an iron-carrying protein according to the claims. The pathogen may comprise *Helicobacter pylori,* wherein the iron-carrying protein comprises human lactoferrin, and wherein the artemisinin-related endoperoxide is selected from the group consisting of artelinate and dihydroartemisinin. The pathogen may comprise *Neisseria meningitidis,* wherein the iron-carrying protein comprises human transferrin, and wherein the artemisinin-related endoperoxide is selected from the group consisting of artelinate and dihydroartemisinin.

The covalent conjugates/compositions may be used in the treatment of *H. pylori* infections by administering to a human or animal subject in need thereof an effective amount of a composition comprising a covalent conjugate between an artemisinin-related endoperoxide and an iron-carrying protein according to the claims.

### DETAILED DESCRIPTION OF THE PREFERRED EMBODIMENT

The invention provides compositions comprising covalent conjugates between artemisinin-related endoperoxides and iron-carrying proteins as described in claim 1. As used herein, the term "covalent conjugate" refers to a compound in which an artemisinin-related endoperoxide is covalently linked to an iron-carrying protein. The term "artemisinin-related endoperoxide" refers to a compound having an endoperoxide bridge, which reacts with an iron atom to form free radicals, causing cell death. Artemisinin-related endoperoxides compounds may also form free radicals in the presence of copper and manganese.

The artemisinin-related endoperoxide of the covalent conjugate of this invention is selected from the group consisting of sesquiterpene lactones and alcohols, carbonates, esters, ethers, and sulfonates thereof, arteflene, 1,2,4-trioxanes, and 1,2,4,5-tetraoxanes. The artemisinin-related endoperoxide may have the structure: 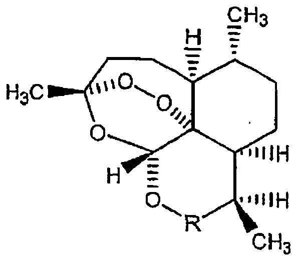 wherein R is 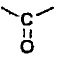 or 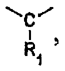 were R₁ is hydrogen, hydroxyl, alkyl, or has the formula: 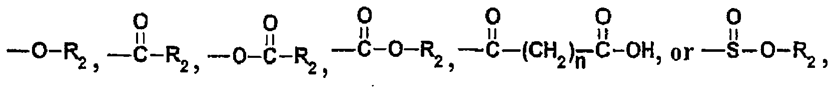 where R₂ is alkyl or aryl and n is 1 to 6, and the pharmaceutically acceptable salts thereof. As used herein, the term "alkyl" means lower alkyl having from 1 to 6 carbon atoms, preferably 1 to 4 carbon atoms. Alkyl groups of the invention may be straightchain or branched-chain groups. The term "aryl" refers to monocyclic and polycyclic aromatic groups containing from 4 to 14 backbone carbon or heteroatoms, and includes both carbocyclic aryl groups and heterocyclic aryl groups. Carbocyclic aryl groups are aryl groups in which all ring atoms are carbon. Heterocyclic aryl groups have from 1 to 4 heteroatoms as ring atoms with the remainder of the ring atoms being carbon. Representative aryl groups include, for example, phenyl and benzyl. Pharmaceutically acceptable salts include the alkali or alkaline earth metal salts, preferably sodium or potassium.

For example, autemisinin-related endoperoxides include artemisinin, where R is 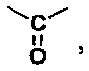 dihydroartemisinin (R₁ = -OH), artesunic acid (R₁ = -OCO(CH₂)₂CO₂H), and artesunate, artemether (R₁= -OCH₃), and arteether (R₁ = -OC₂H₅). Other representative endoperoxide compounds of the invention may include artelinic acid, dihydroartemisinin propyl carbonate, arteflene (Ro. 42-1611) and its analogs (Biirgen et al. (1994) Sixth Int. Cong. Infect. Dis. Abst. 427, p. 152, Prague), 1,2,4-trioxanes (Peters et al. (1993) Ann. Trop. Med. Parasit. 87(1):9-16) and 1,2,4,5-tetraoxanes (Vennerstrometal. (1992) J. Med. Chem. 35(16):3023-3027). Other suitable structural analogs of artemisinin are described in, for example, U.S. Patent Nos. 5,216,175 and 5,180,840; Cumming et al. (1998) J. Med Chem. 41(6):952-64; and PCT patent applications WO 97/01548, WO 99/33461, and WO 00/42046.

The source of artemisinin-related endoperoxides may be natural (*e.g.,* isolated from plants), synthetic, or semi-synthetic. For example, the free radical-generating agents may be produced by expressing the enzymes for the relevant synthetic pathways in a microbial host *(see, e.g.,* Martin et al. (2003) Nature Biotechnol., published online: 1 June 2003, doi:10.1038/nbt833).

As used herein, the term "iron-carrying protein" refers to a protein that is suitable for transporting iron to or into a cell. The iron-carrying protein in the covalent conjugates of the invention may be a mammalian protein, such as a human protein. Examples of iron-carrying proteins include the transferrin family of proteins, neutral gelatinase-associated lipocalin (NGAL) and hemoproteins. Iron plays a vital role in cell growth. The transferrin family of proteins includes transferrin, lactoferrin, ovotransferrin, and melanotransferrin (Aisen & Harris (1989) in Iron Carriers and Iron Proteins (ed. Loehr, VCH, New York) pages 273-320; Baker (1994) Adv. Inorg. Chem. 41:389-463). All these proteins are structurally related single-chain glycoproteins containing 670-690 amino acids. Transferrin transports iron and heme from the circulation into cells. The iron-loaded form of transferrin (holotransferrin) binds to transferrin receptors on the cell surface and is taken inside the cell via receptor-mediated endocytosis, where the iron is released. Lactoferrin has a key role in iron absorption from human milk. Another function of lactoferrin is to withhold iron from infectious agents. Lactoferrin, unlike transferrin, stably binds iron at low pH and is absorbed into the intestine.

NGAL is an iron-carrying protein that is unrelated to the transferrin family of proteins and is proposed to deliver iron to differentiating epithelial cells (Kaplan (2002) Cell 111:603-6). Hemoproteins are proteins such as hemoglobin, myoglobin, hemopexin, cytochromes, catalases, and peroxidases that carry heme as a prosthetic group. For example, hemopexin is a 60 kDa serum glycoprotein that sequesters heme with very high affinity from the blood stream and transports it to the liver (Baker et al. (2003) Proc. Natl. Acad Sci. U.S.A. 100(7):3579-83).

In the covalent conjugates of the invention, the artemisinin-related endoperoxides are linked to the iron-carrying protein in any way that preserves both the activity of the iron-carrying protein and the activity of the artemisinin-related endoperoxide. For example, the artemisinin-related endoperoxide may be linked to a glycosylated iron-carrying protein by first preparing a hydrazide derivative of the artemisinin-related endoperoxide, which is then linked to one or more oxidized polysaccharide groups on the iron-carrying protein, as described in EXAMPLE 1. To make the hydrazide derivative of the artemisinin-related endoperoxide, an ester of the artemisinin-related endoperoxide may first be formed by adding 1-hydroxybenztriazole (HOBt) to the artemisinin-related endoperoxide, followed by addition of ethyl dimethylethylcarbodiimide. The HOBt ester may then be reacted with hydrazine to form the hydrazide derivative. The hydrazide derivative may then be covalently linked to an iron-carrying protein, in which the polysaccharide groups have been oxidized with an oxidizing agent such as sodium periodate. Any artemisinin-related endoperoxide with a -(C=O)NH- group can be used to make a hydrazide derivative that can be linked to transferrin or other iron-carrying glycoproteins *(see, e.g.,* Cumming et al. (1998) J. Med. Chem. 41(6):952-64).

The artemisinin-related endoperoxide may also be linked to a glycosylated iron-carrying protein by first preparing a hydrazine or aminoxy derivative of the artemisinin-related endoperoxide. To make the hydrazine or aminoxy derivative of the artemisinin-related endoperoxide, a halide of the artemisinin-related endoperoxide may first be formed by adding a halo alcohol to the artemisinin-related endoperoxide, followed by addition of boron trifluoride etherate. The halide or the artemisinin-related endoperoxide may then be reacted with hydrazine or hydroxyl amine to form the hydrazine or aminoxy derivative, respectively. The halide group may be substituted by p-toluenesulphonyl or methanesulfonyl group for the reaction. The hydrazine or aminoxy derivative may then be covalently linked to the an iron-carrying protein, in which the polysaccharide groups have been oxidized with an oxidizing agent such as sodium periodate. Any artemisinin-related endoperoxide with a -(C=O)NH- group, a -NH- group, or an -O- group can be used to make either a hydrazine derivative or an aminoxy derivative that can be linked to transferrin or other iron-carrying glycoproteins.

Artemisinin-related endoperoxides that are suitable for linking in this manner include compounds from which a hydrazide, hydrazine, or aminoxy derivative may be prepared without destroying the activity of the endoperoxide bridge. Generally, the hydrazide, hydrazine, or aminoxy group is separated from the endoperoxide bridge by a spacer, such as a hydrocarbon chain. Ethers, esters, amides, sulfides and disulfides may also be used as a spacer. For example, a benzene ring may separate the endoperoxide bridge from the hydrazide, hydrazine, or aminoxy group, as in artelinate. Accordingly, artemisinin-related endoperoxides suitable for use according to the invention include endoperoxides of the structure: 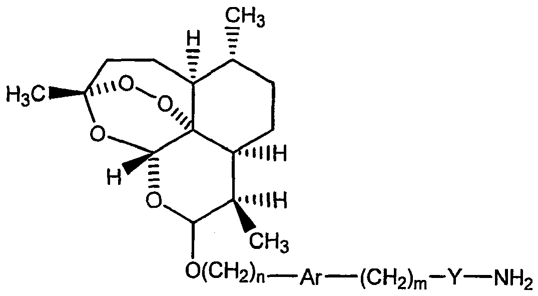 where n = 1-3, m = 0-3, Ar = aryl, for example, phenyl, naphtyl, pyrenyl, pyridyl, or pyrimidinyl, and Y = -(C=O)NH-, -NH-, or -O-. Representative artemisinin-related endoperoxides present in the covalent conjugates of the invention include artelinate and dihydroartemisinin.

Other methods for linking an artemisinin-related endoperoxide to an iron-carrying glycoprotein include borate-mediated linkages to carbohydrate residues on the iron-carrying glycoprotein (*see, e.g.,* U.S. Pat. No. 5,919,708). Thus, artemisinin-related endoperoxides with a borate group will react with an 1,2-diol moiety of carbohydrate residues on the protein surface.

For non-glycosylated iron-carrying proteins, artemisinin-related endoperoxides may be linked to the protein surface by using amino acid side chains, as described in EXAMPLE 2. For example, both artesunate and artelinate have a free carboxylic acid group that can be activated with N-hydroxy succinimide (*see, e.g.,* Lewis et al. (1994) Bioconj. Chem. 5(6):655-76) and a water soluble carbodiimide reagent such as N-etheyl-N'-dimethylaminoethylcarbodiimide to form an active ester. This active ester may be mixed with an aqueous solution of a non-glycosylated iron-carrying protein to link the artemisinin-related endoperoxide to Lys residues on the protein surface.

The covalent conjugates may be purified by using standard methods in the art, for example by using gel-filtration chromatography, ion-exchange, and reverse-phase or hydrophobic interaction High-Pressure Liquid Chromatography (HPLC). The number of molecules of artemisinin-related endoperoxide bound to one molecule of iron-carrying protein may be determined by using standard methods in the art, for example ion-spray mass spectrometry. The ratio of artemisinin-related endoperoxide to iron-carrying proteins in the covalent conjugates will depend on the iron-carrying protein being used and the method of forming the conjugate. For example, covalent conjugates containing between 1 and 10 molecules of the artemisinin-related endoperoxide per molecule of holotransferrin may be obtained using the method described in EXAMPLE 1.

The invention also provides compositions comprising the covalent conjugates of the invention. In some embodiments, the compositions of the invention comprise a covalent conjugate between artelinate and human holotransferrin, as described in EXAMPLE 1. In other embodiments, the compositions comprise a covalent conjugate between artelinate and human hololactoferrin.

The covalent conjugates of the invention may be used in the treatment of cancer. Due to their rapid rate of division, most cancer cells have high rates of iron intake and express higher cell surface concentrations of transferrin receptors than normal cells. It has been shown that administration of iron salts or holotransferrin increases the susceptibility of cancer cells to artemisinin and its analogs (Moore et al. (1995) Cancer Lett. 98:83-7; Singh & Lai (2001) Life Sci. 70:49-56; Sadava et al. (2002) Cancer Lett. 1179:151-6). Thus, the efficacy and selectivity of the artemisinin-related endoperoxides against cancer is increased by covalently linking artemisinin-related endoperoxides to iron-carrying proteins such as holotransferrin because both endoperoxide and iron are transported to or into the same cell at the same time.

The covalent conjugates of the invention may also be used in the treatment of infections by pathogenic organisms that have receptors for the iron-carrying protein in the covalent conjugates. To establish a successful infection, a pathogen must overcome the strict iron limitations imposed by the host. To overcome this limitation, many pathogens obtain iron from iron-carrying host proteins (*see, e.g.,* Cornelissen, (2003) Front. Biosci. 8:D836-47). For example, *Neisseria meningitidis,* the causative agent of bacterial meningitis, expresses cell surface receptors for the iron-carrying proteins transferrin and lactoferrin (Evans & Oakhill (2002) Biochem. Soc. Trans. 30(4):705-7), *Helicobacter pylori,* the etiologic agent of gastritis and peptic ulcer disease in humans, expresses a receptor for human lactoferrin (Husson et al. (1993) Infect. Immun. 61(6):2694-7), and *Staphylococcus aureus* expresses receptors for hemoglobin (Mazmanian et al. (2003) Science 299:906-9) Thus, the covalent conjugates of the invention are useful for killing pathogenic organisms that have receptors for the iron-carrying protein in the conjugate.

For example, a covalent conjugate between an artemisinin-related endoperoxide and human transferrin or human hololactoferrin may be used to treat bacterial meningitis caused by *N. meningitidis* or gastritis and peptic ulcer disease caused by *Helicobacter pylori*.

The covalent conjugates between an artemisinin-related endoperoxide and an iron-carrying protein according to the claims possess superior cytotoxic activities when administered to cancer cells compared to separately administering the artemisinin-related endoperoxide and the iron-carrying protein. In addition, the covalent conjugates of the invention are effective at killing pathogens with receptors for iron- carrying proteins.

Herein described are compositions comprising a covalent conjugate between an artemisinin-related endoperoxide and an iron-carrying protein according to the claims for administration to a subject in need thereof. The covalent conjugates between artemisinin-related endoperoxides and iron-carrying proteins are as described above. For example, some embodiments provide covalent conjugates between artelinate and holotransferrin, as described in EXAMPLE 1.

The methods described herein are applicable to any animal subject, such as a human subject. For example, a subject in need of compositions comprising a covalent conjugate between an artemisinin-related endoperoxides and an iron-carrying protein may be a cancer patient. As described above, rapidly proliferating cells such as cancer cells generally possess higher concentrations of cell surface transferrin receptors. The described methods provide a mechanism for selectively delivering both the endoperoxide moiety and the iron it reacts with to rapidly proliferating cells, such as cancer cells. Accordingly, herein described are compounds according to the claims for use in a method of treating cancer by administering to a human or animal subject in need thereof an effective amount of said compound. Other conditions in which there is hyperproliferation of cells and which the covalent conjugates of the invention may be used to treat include restenosis, autoimmune disease, arthritis, graft rejection, inflammatory bowl disease, or proliferation induced after medical procedures. The described methods comprise administering a compound comprising a covalent conjugate between artelinate and human holotransferrin.

The compounds and compositions comprising a covalent conjugate between an artemisinin-related endoperoxide and an iron-carrying protein according to the claims may also be used for treating an infection by a pathogen expressing cell-surface receptors for the iron- carrying proteins in the covalent conjugate. As used herein, the term "treating an infection by a pathogen" refers to inhibiting the growth of the pathogen and/or preventing or ameliorating the symptoms of disease associated with the infection.

Exemplary pathogens with receptors for iron-carrying host proteins are described above and include *Neisseria meningitidis,* which expresses a receptor for human transferrin and *H. pylori,* which expresses a receptor for human lactoferrin. *S. aureas* has recently been shown to express a receptor for hemoglobin (Mazmanian et al. (2003) Science 299:906-9), and similar proteins are also expressed by *Listeria monocytogenes* and *Bacillus anthracis* (Cabanes et al. (2002) Trends. Microbiol. 10(5):238-45). An exemplary list of pathogens that express receptors for iron-carrying host proteins are shown in Table 1. Once the iron-carrying protein is bound to the receptor expressed by pathogen, the iron or heme is generally released from the iron-carrying protein and transported into the cell *(see, e.g.,* Gray-Owens & Schryvers (1996) Trends Microbiol. 4(5):185-91; Wandersman & Stojiljkovie (2000) Curr. Op. Microbiol. 3:215-20).

**Table 1. Receptors for Iron-Carrying Proteins in Human and Animal Pathogens**

| Pathogen | Host | Disease | Receptor |
|---|---|---|---|
| *Moraxella bovis* | Bovine | Kerato-conjunctivitis | Transferrin, lactoferrin¹ |
| *Moraxella catarrhalis* | Human | Otitis media | Transferrin, lactoferrin¹ |
| *Moraxella lacunata* | Human | Kerato-conjunctivitis | Transferrin, lactoferrin¹ |
| *Neisseria meningitidis* | Human | Meningitis | Trausferrin, lactoferrin, hemoglobin^{1,19} |
| *Neisseria gonorrheae* | Human | Gonorrhea | Transferrin, lactoferrin, hemoglobin^{1,18} |
| *Actinobacillus actinomycetecomitans* | Human | Juvenile periodontitis | Transferrin¹ |
| *Actinobacillus equuli* | Equine | Septicemia | Transferrin¹ |
| *Actinobacillus pleuropneumoniae* | Porcine | Pneumonia | Transferrin¹ |
| *Haemophilus agnii* | Ovine | Septicemia | Transferrin¹ |
| *Haemophilus avium* | Poultry | Sinusitis | Transferrin¹ |
| *Haemophilus influenzae* | Human | Meningitis, otitis media | Transferrin, hemoglobin^{1,20} |
| *Haemophilus paragallinarum* | Poultry | Infectious coryza | Transferrin¹ |
| *Haemophilus somnus* | Bovine | Thromboembolic meningoencephalitis | Transferrin¹ |
| *Haemophilus parasuis* | Porcine | Glasser's disease | Transferrin¹ |
| *Haemophilus ducreyi* | Human | Genital ulcer disease | Hemoglobin¹⁵ |
| *Pasteurella haemolytica* | Bovine, ovine, caprine | Shipping fever, pasteurellosis | Transferrin¹ |
| *Pasteurella multocida* | Bovine | Pneumonia, septicemia | Transferrin¹ |
| *Staphylococcus aureus* | Human | Bacteremia, pneumonia, endocarditis, septic arthritis, osteomyelitis, deep abscesses, food poisoning | Transferrin, hemoglobin^{2,13} |
| *Staphylococcus epidermidis* | Human | Endocarditis, endopthalmitis, septicemia, cystitis | Transferrin² |
| *Streptococcus pneumoniae* | Human | Pneumonia, meningitis, bacteremia, otitis media | Lactoferrin³ |
| *Leishmania chagasi* | Human | Leishmaniasis | Transferrin, lactoferrin⁴ |
| *Escherichia coli K88* | Porcine | Enteropathogenesis | Transferrin⁵ |
| *Tritrichonomas foetus* | Cattle | Trichomoniasis | Lactoferrin, hemoglobin⁶, |
| *Treponema pallidum* | Human | Syphilis | Lactoferrin⁷ |
| *Mycoplasma pneumonia* | Human | Pneumonia | Lactoferrin⁸ |
| *Bordetella pertussis* | Human | Whooping cough | Lactoferrin⁹ |
| *Trichonomas vaginalis* | Human | Vaginosis | Lactoferrin¹⁰ |
| *Aeromonas salmonicida* | Fish | Furunculosis | Transferrin, lactoferrin¹¹ |
| *Helicobacter pylori* | Human | Gastritis, gastric and duodenal ulcers, gastric adenocarcinoma, lymphoma | Lactoferrin¹² |
| *Yersinia enterocolitica* | Human | Enteritis | Hemoglobin, myoglobin, hemopexin, catalase, albumin-heme¹⁴ |
| *Vibrio vulnificus* | Eel | Food poisoning, septicemia, wound infections | Hemoglobin¹⁶ |
| *Porphyromonas gingivalis* | Human | Periodontal disease | Hemoglobin¹⁷ |

| | | | |
|---|---|---|---|
| 1 Gray-Owen & Schryvers (1996) Trends Microbiol. 4(5):185-91 2 Modun et al. (1998) Infect. Immun. 66(8):3591-6 3 Hammerschmidt et al. (1999) Infect. Immun. 67(4):1683-7 4 Britigan et al. (1998) Adv. Exp. Med. Biol. 443: 135-40 5 Grange et al. (1997) Adv. Exp. Med Biol. 412:357-61 6 Tachezy et al. (1996) Exp. Parasitol. 83(2):216-28 7 Alderete et al. (1988) Genitourin. Med. 64(6):359-63 8 Tryon & Baseman (1987) Microb. Pathog. 3(6):437-43 9 Redhead et al. (1987) J. Gen. Microbiol. 133(4):891-8 10 Peterson & Alderete (1984) J. Exp. Med. 160(2):398-410 11 Chart & Trust (1983) J. Bacteriol. 156(2):758-64 12 Dhaenens et al. (1997) Infect. Immun. 65(2):514-8 13 Mazmanian et al. (2003) Science 299:906-9 14 Bracken et al. (1999) J. Bacteriol. 181(19):6063-72 15 Al-Twafiq et al. (2000) J. Infect. Dis. 181(3):1049-54 16 Fouz et al. (1997) Microbiol. Lett. 156(2):187-91 17 Simpson et al. (2000) J. Bacteriol. 182(10): 5737-48 18 Chen et al. (1996) Infect. Immun. 64:5008-14 19 Stojiljkovic et al. (1996) J. Bacteriol. 179(15):4670-78 20 Frangipane et al. (1994) FEMS Microbiol. Lett. 118:243-8 | | | |

The methods described herein provide a mechanism for selectively delivering an endoperoxide moiety and the iron it reacts with directly to the cell membrane of a pathogenic organisms by binding of the covalent conjugate to a receptor for iron-carrying protein. According to the described methods, the endoperoxide moiety in the bound covalent conjugate reacts with the iron or heme released from the iron-carrying protein, producing harmful free radicals in close proximity to the pathogen. Accordingly, described herein are compositions according to the claims for use in a method of treating a disease caused by *Helicobacter pylori* by administering an effective amount of said composition comprising a covalent conjugate between an artemisinin-related endoperoxide and human hololactoferrin to a human subject in need thereof. Also described herein are compositions according to the claims for use in a method of for treating a disease caused by *Neisseria meningitidis* by administering an effective amount of said composition comprising a covalent conjugate between an artemisinin-related endoperoxide and human holotransferrin to a human subject in need thereof.

Other exemplary infections that an effective amount of a composition comprising a covalent conjugate of the invention may be used to treat include topical bacterial infections, such as gingivitis, skin, and eye infections.

Effective amounts of the covalent conjugates will generally range up to the maximally tolerated dosage, but the concentrations are not critical and may vary widely. The precise amounts employed by the attending physician will vary, of course, depending on the compound, route of administration, physical condition of the patient and other factors. The daily dosage may be administered as a single dosage or may be divided into multiple doses for administration.

In the methods described herein, the amount of the conjugates of the invention actually administered will be a therapeutically effective amount, which term is used herein to denote the amount needed to produce a substantial beneficial effect. Effective doses may be extrapolated from dose- response curves derived from *in vitro* or animal model test systems. The animal model is also typically used to determine a desirable dosage range and route of administration. Such information can then be used to determine useful doses and routes for administration in humans or other mammals. The determination of an effective dose is well within the capability of those skilled in the art. Thus, the amount actually administered will be dependent upon the individual to which treatment is to be applied, and will preferably be an optimized amount such that the desired effect is achieved without significant side-effects.

Therapeutic efficacy and possible toxicity of the covalent conjugates of the invention may be determined by standard pharmaceutical procedures, in cell cultures or experimental animals (*e.g*, ED₅₀, the dose therapeutically effective in 50% of the population; and LD₅₀, the dose lethal to 50% of the population). The dose ratio between therapeutic and toxic effects is the therapeutic index, and it can be expressed as the ratio

LD₅₀ to ED₅₀. Covalent conjugates that exhibit large therapeutic indices are particularly suitable in the practice of the methods described herein. The data obtained from cell culture assays and animal studies may be used in formulating a range of dosage for use in humans or other mammals. The dosage of such conjugates lies preferably within a range of circulating concentrations that include the ED₅₀ with little or no toxicity. The dosage typically varies within this range depending upon the dosage form employed, sensitivity of the patient, and the route of administration. Thus, optimal amounts will vary with the method of administration, and will generally be in accordance with the amounts of conventional medicaments administered in the same or a similar form.

The covalent conjugates of the invention may be used for treatment by being administered alone, or in combination with one or more additional therapeutically active agents. For example, in the treatment of cancer, the conjugates may be administered in combination with therapeutic agents including androgen inhibitors, such as flutamide and luprolide ; antiestrogens, such as tomoxifen; antimetabolites and cytotoxic agents, such as daunorubicin, fluorouracil, floxuridine, interferon alpha, methotrexate, plicamycin, mecaptopurine, thioguanine, adriamycin, carmustine, lomustine, cytarabine, cyclophosphamide, doxorubicin, estramustine, altretamine, hydroxyurea, ifosfamide, procarbazine, mutamycin, busulfan, mitoxantrone, carboplatin, cisplatin, streptozocin, bleomycin, dactinomycin, and idamycin; hormones, such as medroxyprogesterone, estramustine, ethinyl estradiol, estradiol, leuprolide, megestrol, octreotide, diethylstilbestrol, chlorotrianisene, etoposide, podophyllotoxin, and goserelin ; nitrogen mustard derivatives, such as melphalan, chlorambucil, methlorethamine, and thiotepa, steroids, such as betamethasone; and other antineoplastic agents, such as live *Mycobacterium bovis,* dicarbazine, asparaginase, leucovorin, mitotane, vincristine, vinblastine, and taxotere. Appropriate amounts in each case will vary with the particular agent, and will be either readily known to those skilled in the art or readily determinable by routine experimentation.

The covalent conjugates of the invention may also be used for treatment by being administered in combination with an agent that increases iron transport into cells, for example, by increasing the cell surface number of receptors for the iron-carrying protein in the conjugate. It has been shown for example, that insulin, insulin-like growth factor I, and epidermal growth factor cause an increase in the number of transferrin receptors at the cells surface (*see, e.g.,* Davis et al. (1987) J. Biol. Chem. 261(19):8708-11; Davis et al. (1986) J. Biol. Chem. 262(17):13126-34). Therefore, in some cases, covalent conjugates of the invention containing transferrin are administered in combination with insulin, insulin-like growth factor I, or epidermal growth factor.

Administration of the covalent conjugates of the invention is accomplished by any effective route, *e.g.,* parenterally or orally. Methods of administration include topical (for examples, skin patches), inhalational, buccal, intraarterial, subcutaneous, intramedullary, intravenous, intranasal, intrarectal, intraocular administration, and other conventional means. For example, the covalent conjugates for use in treatment may be injected directly into a tumor, into the vicinity of a tumor, or into a blood vessel that supplies blood to the tumor.

The covalent conjugates of the invention may be formulated into a composition that additionally comprises suitable pharmaceutically acceptable carriers, including excipients and other compounds that facilitate administration of the covalent conjugate to a mammalian subject. Further details on techniques for formulation and administration may be found in the latest edition of "Remington's Pharmaceutical Sciences" (Maack Publishing Co, Easton PA).

Compositions for oral administration may be formulated using pharmaceutically acceptable carriers well known in the art, in dosages suitable for oral administration. Such carriers enable the compositions containing covalent conjugates of the invention to be formulated as tablets, pills, dragees, capsules, liquids, gels, syrups, slurries, suspensions, etc. , suitable for ingestion by a subject. Compositions for oral use may be formulated, for example, in combination with a solid excipient, optionally grinding the resulting mixture, and processing the mixture of granules, after adding suitable additional compounds, if desired, to obtain tablets or dragee cores. Suitable excipients include carbohydrate or protein fillers. These include sugars, including lactose, sucrose, mannitol, or sorbitol, starch from corn, wheat, rice, potato, or other plants ; cellulose such as methyl cellulose, hydroxypropylmethyl-cellulose, or sodium carboxymethylcellulose; and gums including arabic and tragacanth ; as well as proteins, such as gelatin and collagen. If desired, disintegrating or solubilising agents may be added, such as the crosslinked polyvinyl pyrrolidone, agar, alginic acid, or a salt thereof, such as sodium alginate.

Dragee cores are provided with suitable coatings such as concentrated sugar solutions, which may also contain gum arabic, talc, polyvinylpyrrolidone, carbopol gel, polyethylene glycol, and/or titanium dioxide, lacquer solutions, and suitable organic solvents or solvent mixtures. Dyestuffs or pigments may be added to the tablets or dragee coatings for product identification or to characterize the quantity of active compound (*i.e.,* dosage).

Covalent conjugates for oral administration may be formulated, for example, as push-fit capsules made of gelatin, as well as soft, sealed capsules made of gelatin and a coating such as glycerol or sorbitol. Push-fit capsules may contain covalent conjugates mixed with filler or binders such as lactose or starches, lubricants such as talc or magnesium stearate, and, optionally, stabilizers. In soft capsules, the covalent conjugates may be dissolved or suspended in suitable liquids, such as fatty oils, liquid paraffin, or liquid polyethylene glycol with or without stabilizers.

Compositions for parenteral administration include aqueous solutions of one or more covalent conjugates of the invention. For injection, the covalent conjugates may be formulated in aqueous solutions, such as in physiologically compatible buffers such as Hank's solution, Ringer's solution, or physiologically buffered saline. Aqueous injection suspensions may contain substances, which increase the viscosity of the suspension, such as sodium carboxymethyl cellulose, sorbitol, or dextran. Additionally, suspensions of the covalent conjugates may be prepared as appropriate oily injection suspensions. Suitable lipophilic solvents or vehicles include fatty oils such as sesame oil, or synthetic fatty acid esters, such as ethyl oleate or triglycerides, or liposomes. Optionally, the suspension may also contain suitable stabilizers or agents, which increase the solubility of the covalent conjugate to allow for the preparation of highly concentrated solutions.

For topical or nasal administration, penetrants appropriate to the particular barrier to be permeated are typically used in the formulation. Examples of these are 2-pyrrolidone, N-methyl-2-pyrrolidone, dimethylacetamide, dimethyl-formamide, propylene glycol, methyl or isopropyl alcohol, dimethyl sulfoxide, and azone. Additional agents may further be included to make the formulation cosmetically acceptable. Examples of these are fats, waxes, oils, dyes, fragrances, preservatives, stabilizers, and surface-active agents. Keratolytic agents such as those known in the art may also be included. Examples are salicylic acid and sulfur. For topical administration, the composition may be in the form of a transdermal ointment or patch for systemic delivery of the compound and may be prepared in a conventional manner (*see, e.g.,* Barry, Dermatological Formulations (Drugs and the Pharmaceutical Sciences--Dekker); Harrys Cosmeticology (Leonard Hill Books).

For rectal administration, the compositions for use in treatment may be administered in the form of suppositories or retention enemas. Such compositions may be prepared by mixing the covalent conjugates with a suitable non-irritating excipient that is solid at ordinary temperatures but liquid at the rectal temperature and will therefore melt in the rectum to release the drug. Suitable excipients include, but are not limited to, cocoa butter and polyethylene glycols.

The amounts of each of these various types of additives will be readily apparent to those skilled in the art, optimal amounts being the same as in other, known formulations designed for the same type of administration. Stratum corneum penetration enhancers, for example, will typically be included at levels within the range of about 0.1% to about 15%.

Compositions containing the covalent conjugates of the present invention may be manufactured in a manner similar to that known in the art (*e.g*., by means of conventional mixing, dissolving, granulating, dragee-making, levigating, emulsifying, encapsulating, entrapping or lyophilizing processes). The compositions may also be modified to provide appropriate release characteristics, *e.g.*, sustained release or targeted release, by conventional means (*e.g*., coating).

Compositions containing the covalent conjugates may be provided as a salt and can be formed with many acids, including hydrochloric, sulfuric, acetic, lactic, tartaric, malic, succinic. Salts tend to be more soluble in aqueous or other protonic solvents than are the corresponding free base forms.

After compositions formulated to contain covalent conjugates and an acceptable carrier have been prepared, they can be placed in an appropriate container and labeled for use.

### EXAMPLE 1

This Example describes a method for making a representative composition of the invention containing one or more artemisinin-related endoperoxide molecules covalently linked to a molecule of holotransferrin.

*Synthesis of Artelinic Acid Hydrazide (ART-NH-NH₂):* Attempts to prepare an hydrazide derivative of artesunate were unsuccessful and resulted in the formation of dihydroartemisinin due to the cyclization reaction. Artelinate was synthesized from dihydroartemisinin as previously described (Shrimali et al. (1998) Indian J. Chem. 37B:1161-1163). Artelinic acid (0.1 g, 0.24 mmol) was dissolved in anhydrous acetonitrile (0.48 mL). To this solution, 1-hydroxybenztriazole (HOBt) (0.038 g, 0.29 mmol) was added, followed by addition of ethyl dimethylethylcarbodiimide (0.055 g, 0.29 mmol). The reaction mixture was stirred at room temperature and monitored by thin-layer chromatography (TLC) until all the acid was converted to the HOBt ester.

A solution of hydrazine (0.46 mL, 0.48 mmol) in acetonitrile (0.48 mL) was cooled to 0°C, and the above reaction mixture was added to it while maintaining the temperature between 0-10°C. The reaction was complete in 10 minutes, as measured by TLC. The reaction mixture was poured into water (5 mL), extracted with ethylacetate (3 x 10 mL), and washed with brine. The organic phase was separated, dried over anhydrous sodium sulfate, and evaporated to dryness. The crude product was purified by silica gel chromatography using a gradient of methanol/chloroform to give the pure product (0.078 g) in 76% yield.

*Synthesis of Artelinate-Holotransferrin Conjugate:* Holotransferrin (2 mg), dissolved in 1 ml of 0.1 mol/L sodium acetate pH 5.5, was oxidized at a level of its glycans, at room temperature for 30 minutes with 10 mmol/L sodium periodate. The solution was applied to a short Sephadex G-25 column equipped with a UV monitor. The column was eluted with 0.1 mol/L sodium acetate pH 5.5, and the protein fractions were collected. A solution of excess ART-NH-NH2 was added to the oxidized holotransferrin, and the reaction was kept overnight at room temperature with gentle shaking. The artelinate-holotransferrin conjugate solution was then applied to a Sephadex G-25 column to remove excess ART-NH-NH2. The column was eluted with 0.1 mol/L Tris-HCl buffer, pH 7.5 and the protein fractions were collected. The conjugate was stored at 4°C.

The artelinate-holotransferrin conjugate was purified by hydrophobic interaction HPLC to obtain a homogenous protein conjugate. The number of artelinate molecules per molecule of holotransferrin was determined by ion-spray mass spectrometry.

### EXAMPLE 2

This Example describes a method for making a representative composition of the invention containing one or more artemisinin-related endoperoxide molecules covalently linked to a molecule of hemoglobin.

For covalent modification of non-glycosylated proteins such as hemoglobin with artemisinin derivatives, carboxylic acid derivatives of artemisinin such as artelinic acid is first activated as an N-hydroxy succinimide (HOSu) ester to react with lysine residues on the protein surface. Artelinic acid (4.2 mg, 0.01 mmol) is dissolved in dimethylformamide (DMF) (0.5 mL), and the solution is cooled in ice-bath. To this solution, N-ethyl-N'-dimethlyaminoethyl carbodiimide (EDC) (1.5 mg, 0.01 mmol) and HOSu (1.1 mg, 0.01 mmol) are added. The reaction mixture is kept stirring for 2 hours at 0°C to complete the formation of the HOSu ester of artelinic acid.

Hemoglobin (10 mg) is dissolved in 0.1 M phosphate buffer 7.0 (5 mL). The DMF solution of the artelinic acid HOSu ester is slowly added with stirring at 0°C to the solution of hemoglobin. The reaction mixture is kept stirring for 2 hours at 0°C, and for another 2 hours at room temperature. The reaction mixture is then applied to the Sephadex G-25 column which is equilibrated with 0.1 M phosphate buffer pH 7, and the column is eluted with the same buffer. The modified hemoglobin elutes at the void volume. The fractions containing hemoglobin are combined, and then purified by hydrophobic interaction HPLC. The number of artemisinin derivatives attached to the protein is determined by ion spray mass spectrometry.

## Claims

1. A covalent conjugate consisting of an artemisinin-related endoperoxide selected from the group consisting of sesquiterpene lactones and alcohols, carbonates, esters, ethers, and sulfonates thereof, arteflene, 1,2,4-trioxanes, and 1,2,4,5-tetraoxanes covalently conjugated to an iron-carrying protein selected from the group consisting of transferrin, holotransferrin, lactoferrin, hololactoferrin, haemoglobin, hemopexin, and neutral gelatinase-associated lipocalin, wherein the iron-carrying protein releases iron that reacts with an endoperoxide bridge on the endoperoxide to form free radicals.

2. The covalent conjugate of claim 1 wherein the iron-carrying protein is a mammalian protein.

3. The covalent conjugate of claim 2 wherein the iron-carrying protein is a human protein.

4. The covalent conjugate of claim 1 wherein the iron-carrying protein is holotransferrin or hololactoferrin.

5. The covalent conjugate of any one of claims 1 to 4, wherein the endoperoxide comprises artelinate or is a hydrazide derivative.

6. The covalent conjugate of any one of claims 1 to 4, wherein the endoperoxide has the structure: 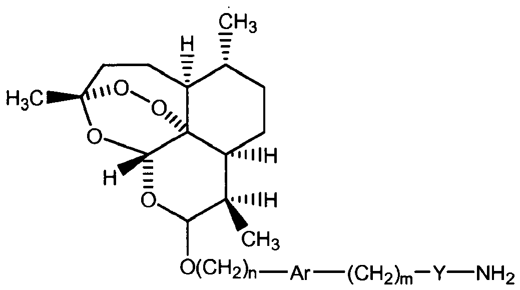 where n = 1-3, m = 0-3, Ar = aryl, and Y = -(C=O)NH-, -NH-, or -O-.

7. The covalent conjugate of any one of claims 1 to 4, wherein the endoperoxide is artelinate and the iron-carrying protein is holotransferrin, the endoperoxide is artelinate and the iron-carrying protein is hololactoferrin or the endoperoxide is artelinate and the iron-carrying protein is haemoglobin.

8. A composition comprising a covalent conjugate of any one of claims 1 to 7 and a pharmaceutically acceptable carrier.

9. A covalent conjugate as defined in any one of claims 1 to 7 for use in the treatment of cancer, e.g., in a human subject, optionally by parenteral administration.

10. The covalent conjugate for use as claimed in claim 9, wherein the endoperoxide is selected from the group consisting of artelinate and dihydroartemisinin and/or wherein the iron-carrying protein is holotransferrin.

11. A covalent conjugate as defined in any one of claims 1 to 7 for use in the treatment of an infection by a pathogen, wherein the pathogen expresses receptors for the iron-carrying protein.

12. The covalent conjugate for use as claimed in claim 11, wherein the endoperoxide is selected from the group consisting of artelinate and dihydroartemisinin and/or wherein the iron-carrying protein is selected from the group consisting of holotransferrin, hololactoferrin, haemoglobin, hemopexin, and neutral gelatinase-associated lipocalin.

13. The covalent conjugate for use as claimed in claim 11 wherein either the pathogen comprises *Helicobacter pylori,* the iron-carrying protein comprises human lactoferrin, and the endoperoxide is artelinate or dihydroartemisinin; or wherein the pathogen comprises *Neisseria meningitidis,* the iron-carrying protein comprises human transferrin, and the artemisinin-related endoperoxide is artelinate or dihydroartemisinin.

14. A covalent conjugate of any one of claims 1 to 7 for use as a pharmaceutical.

15. The covalent conjugate of any one of claims 1 to 4, wherein the endoperoxide is artemisinin, dihydroartemisinin, artelinate, artesunic acid, artesunate, artemether, arteether, artelinic acid, dihydroartemisinin propyl carbonate, or arteflene.

16. The composition of claim 8, wherein the endoperoxide is artemisinin, dihydroartemisinin, artelinate, artesunic acid, artesunate, artemether, arteether, artelinic acid, dihydroartemisinin propyl carbonate, or arteflene.

## Patentansprüche

1. Ein kovalentes Konjugat, bestehend aus einem mit Artemisinin verwandten Endoperoxid, das ausgewählt ist aus der Gruppe, bestehend aus Sesquiterpen-Lactonen und Alkoholen, Carbonaten, Estern, Ethern und Sulfonaten von diesen, Arteflen, 1,2,4-Trioxanen und 1,2,4,5-Tetraoxanen, die kovalent mit einem Eisen tragenden Protein konjugiert sind, das ausgewählt ist aus der Gruppe, bestehend aus Transferrin, Holotransferrin, Lactoferrin, Hololactoferrin, Hämoglobin, Hemopexin und mit neutraler Gelatinase assoziiertem Lipocalin, wobei das Eisen tragende Protein Eisen freisetzt, das mit einer Endoperoxidbrücke auf dem Endoperoxid reagiert, um freie Radikale zu bilden.

2. Das kovalente Konjugat nach Anspruch 1, wobei das Eisen tragende Protein ein Säugerprotein ist.

3. Das kovalente Konjugat nach Anspruch 2, wobei das Eisen tragende Protein ein menschliches Protein ist.

4. Das kovalente Konjugat nach Anspruch 1, wobei das Eisen tragende Protein Holotransferrin oder Hololactoferrin ist.

5. Das kovalente Konjugat nach irgendeinem der Ansprüche 1 bis 4, wobei das Endoperoxid Artelinat umfasst oder ein Hydrazidderivat ist.

6. Das kovalente Konjugat nach irgendeinem der Ansprüche 1 bis 4, wobei das Endoperoxid die folgende Struktur aufweist: 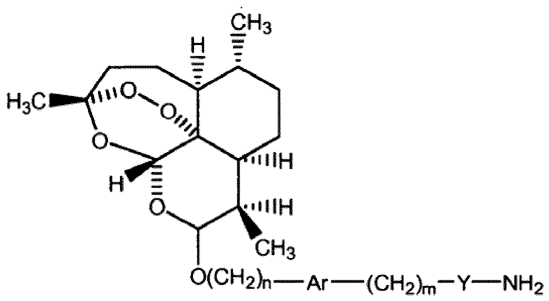 wobei n = 1 - 3, m = 0 - 3, Ar = Aryl und Y = -(C=O)NH-, -NH- oder -O-.

7. Das kovalente Konjugat nach irgendeinem der Ansprüche 1 bis 4, wobei das Endoperoxid Artelinat ist und das Eisen tragende Protein Holotransferrin ist, das Endoperoxid Artelinat ist und das Eisen tragende Protein Hololactoferrin ist oder das Endoperoxid Artelinat ist und das Eisen tragende Protein Hämoglobin ist.

8. Eine Zusammensetzung, welche ein kovalentes Konjugat nach irgendeinem der Ansprüche 1 bis 7 und einen pharmazeutisch verträglichen Träger umfasst.

9. Ein kovalentes Konjugat wie in irgendeinem der Ansprüche 1 bis 7 definiert zur Verwerdung bei der Behandlung von Krebs, z.B. bei einem menschlichen Patienten, gegebenenfalls durch parenterale Verabreichung.

10. Das kovalente Konjugat zur Verwendung wie in Anspruch 9 beansprucht, wobei das Endoperoxid ausgewählt ist aus der Gruppe, bestehend aus Artelinat und Dihydroartemisinin, und/oder wobei das Eisen tragende Protein Holotransferrin ist.

11. Ein kovalentes Konjugat wie in irgendeinem der Ansprüche 1 bis 7 definiert zur Verwerdung bei der Behandlung einer Infektion mit einem Pathogen, wobei das Pathogen Rezeptoren für das Eisen tragende Protein exprimiert.

12. Das kovalente Konjugat zur Verwerdung wie in Anspruch 11 beansprucht, wobei das Endoperoxid ausgewählt ist aus der Gruppe, bestehend aus Artelinat und Dihydroartemisinin, und/oder wobei das Eisen tragende Protein ausgewählt ist aus der Gruppe, bestehend aus Holotransferrin, Hololactoferrin, Hämoglobin, Hemopexin und mit neutraler Gelatinase assoziiertem Lipocalin.

13. Das kovalente Konjugat zur Verwerdung wie in Anspruch 11 beansprucht, wobei entweder das Pathogen *Helicobacter pylori* umfasst, das Eisen tragende Protein menschliches Lactoferrin umfasst und das Endoperoxid Artelinat oder Dihydroartemisinin ist; oder wobei das Pathogen *Neisseria meningiti*dis umfasst, das Eisen tragende Protein menschliches Transferrin umfasst und das mit Artemisinin verwandte Endoperoxid Artelinat oder Dihydroartemisinin ist.

14. Ein kovalentes Konjugat nach irgendeinem der Ansprüche 1 bis 7 zur Verwendung als ein Pharmazeutikum.

15. Das kovalente Konjugat nach irgendeinem der Ansprüche 1 bis 4, wobei das Endoperoxid Artemisinin, Dihydroartemisinin, Artelinat, Artesunsäure, Artesunat, Artemether, Arteether, Artelinsäure, Dihydroartemisininpropylcarbonat oder Arteflen ist.

16. Die Zusammensetzung nach Anspruch 8, wobei das Endoperoxid Artemisinin, Dihydroartemisinin, Artelinat, Artesunsäure, Artesunat, Artemether, Arteether, Artelinsäure, Dihydroartemisininpropylcarbonat oder Arteflen ist.

## Revendications

1. Conjugué covalent consistant en un endoperoxyde associé à l'artémisinine sélectionné dans le groupe consistant en des lactones et des alcools sesquiterpéniques, des carbonates, des esters, des éthers, et des sulfonates de ceux-ci, l'artéflène, des 1,2,4-trioxanes, et des 1,2,4,5-tétraoxanes conjugués de façon covalente à une protéine de transport du fer sélectionnée dans le groupe consistant en la transferrine, l'holotransferrine, la lactoferrine, l'hololactoferrine, l'hémoglobine, l'hémopexine, et la lipocaline associée à la gélatinase des neutrophiles, où la protéine de transport du fer libère du fer qui réagit avec un pont endoperoxyde sur l'endoperoxyde afin de former des radicaux libres.

2. Conjugué covalent selon la revendication 1, dans lequel la protéine de transport du fer est une protéine mammalienne.

3. Conjugué covalent selon la revendication 2, dans lequel la protéine de transport du fer est une protéine humaine.

4. Conjugué covalent selon la revendication 1, dans lequel la protéine de transport du fer est l'holotransferrine ou l'hololactoferrine.

5. Conjugué covalent selon l'une quelconque des revendications 1 à 4, dans lequel l'endoperoxyde comprend de l'artélinate ou est un dérivé d' hydrazide.

6. Conjugué covalent selon l'une quelconque des revendications 1 à 4, dans lequel l'endoperoxyde possède la structure : 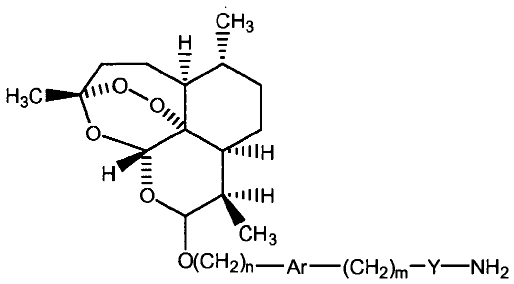 où n = 1-3, m = 0-3, Ar = aryle, et Y = -(C=O)NH-, -NH-, ou -O-.

7. Conjugué covalent selon l'une quelconque des revendications 1 à 4, dans lequel l'endoperoxyde est l'artélinate et la protéine de transport du fer est l'holotransferrine, l'endoperoxyde est l'artélinate et la protéine de transport du fer est l'hololactoferrine ou l'endoperoxyde est l'artélinate et la protéine de transport du fer est l'hémoglobine.

8. Composition comprenant un conjugué covalent selon l'une quelconque des revendications 1 à 7, et un support pharmaceutiquement acceptable.

9. Conjugué covalent tel que défini dans l'une quelconque des revendications 1 à 7, destiné à être utilisé dans le traitement du cancer, par exemple chez un sujet humain, facultativement par une administration parentérale.

10. Conjugué covalent destiné à être utilisé selon la revendication 9, dans lequel l'endoperoxyde est sélectionné dans le groupe consistant en l'artélinate et la dihydroartémisinine et/ou dans lequel la protéine de transport du fer est l'holotransferrine.

11. Conjugué covalent tel que défini dans l'une quelconque des revendications 1 à 7, destiné à être utilisé dans le traitement d'une infection par un agent pathogène, où l'agent pathogène exprime des récepteurs pour la protéine de transport du fer.

12. Conjugué covalent destiné à être utilisé selon la revendication 11, dans lequel l'endoperoxyde est sélectionné dans le groupe consistant en l'artélinate et la dihydroartémisinine et/ou dans lequel la protéine de transport du fer est sélectionnée dans le groupe consistant en l'holotransferrine, l'hololactoferrine, l'hémoglobine, l'hémopexine, et la lipocaline associée à la gélatinase des neutrophiles.

13. Conjugué covalent destiné à être utilisé selon la revendication 11, dans lequel soit l'agent pathogène comprend *Helicobacter pylori,* la protéine de transport du fer comprend la lactoferrine humaine, et l'endoperoxyde est l'artélinate ou la dihydroartémisinine ; ou dans lequel l'agent pathogène comprend *Neisseria meningitidis,* la protéine de transport du fer comprend la transferrine humaine, et l'endoperoxyde associé à l'artémisinine est l'artélinate ou la dihydroartémisinine.

14. Conjugué covalent selon l'une quelconque des revendications 1 à 7, destiné à être utilisé en tant qu'agent pharmaceutique.

15. Conjugué covalent selon l'une quelconque des revendications 1 à 4, dans lequel l'endoperoxyde est l'artémisinine, la dihydroartémisinine, l'artélinate, l'acide artésunique, l'artésunate, l'artéméther, l'artééther, l'acide artélinique, le propylcarbonate de dihydroartémisinine, ou l'artéflène.

16. Composition selon la revendication 8, dans laquelle l'endoperoxyde est l'artémisinine, la dihydroartémisinine, l'artélinate, l'acide artésunique, l'artésunate, l'artéméther, l'artééther, l'acide artélinique, le propylcarbonate de dihydroartémisinine, ou l'artéflène.
